# EUROPÄISCHE PATENTSCHRIFT

(11) **EP 0 563 602 B1**
(45) Veröffentlichungstag und Bekanntmachung des Hinweises auf die Patenterteilung: **27.12.1996**
(21) Anmeldenummer: 93103400.3
(22) Anmeldetag: 03.03.1993
(51) Int. Cl.: A61F 2/16

(54) **Korrekturlinse, welche auf die Vorderseite einer natürlichen Linse eines Auges implantierbar ist**
Correction lens implantable on the front of a natural lens
Lentille de correction implantable sur la partie frontale du cristallin

(30) Priorität: 03.04.1992 DE 4211265
(43) Veröffentlichungstag der Anmeldung: 06.10.1993
(73) Patentinhaber: Chiron Adatomed Pharmazeutische und Medizintechnische Gesellschaft mbH, 85609 Dornach (DE)
(72) Erfinder: Fedorov, Svyatoslav N., Prof. Eye Microsurgery, Beskudnikovsky blod 59A Moskau 127986 (SU); Zuev, Victor K. Eye Microsurgery intersectoral, blod 59 A Moskau 127986 (SU); Fechner, Paul U., Dr., W-3000 Hannover (DE); Kreiner, Christine F., Dr., W-8000 München 82 (DE); Serester, Alexander, W-8000 München 82 (DE)
(74) Vertreter: Nöth, Heinz, Dipl.-Phys.

(56) Entgegenhaltungen:
- EP-A- 0 375 176
- FR-A- 1 103 399
- US-A- 4 769 035

## Beschreibung

Die Erfindung betrifft eine Korrekturlinse nach dem Oberbegriff des Patentanspruches 1.

Eine derartige, aus der US-A-4,769,035 bekannte Korrekturlinse kann zur Behandlung von Myopie, Hyperopie, Astigmatismus zum Einsatz gebracht werden. Die Linse wird dabei durch Implantation auf die Vorderseite der natürlichen Augenlinse aufgesetzt. Die Korrekturlinse besitzt um den normalerweise kreisrund ausgebildeten optischen Linsenteil eine sich daran anschließende Haptik. Bei der Implantation kommen die Haptikteile zwischen Iris und vorderer Oberfläche der natürlichen Augenlinse und den Zonula-Fasern zu liegen, wobei eine Scheuerbelastung auf die Zonula-Fasern, die sich zwischen der natürlichen Augenlinse und dem ziliaren Muskel erstrecken, bei der natürlichen Augenlinsenbewegung vermieden wird. Die Haptikteile erstrecken sich dabei gegenüber der optischen Linsenachse schräg nach vorne und drücken die Korrekturlinse mit ihrer Rückseite auf die Vordefläche der natürlichen Augenlinse.

Die implantierte Linse dient als Ersatz für herkömmliche Brillengläser, auf die Kornea aufgesetzte Kontaktlinsen oder andere Korrekturverfahren, wie Entfernen von Korneaschichten und anderen Techniken.

Aus der EP-A-0 375 176 ist eine Intraokularlinse bekannt, welche nach einer Kataraktoperation anstelle der natürlichen Augenlinse in den verbliebenen Teil des Augenkapselsackes eingesetzt wird. Die Haptik stützt sich im implantierten Zustand an der Innsenseite der Peripherie des Kapselsackes an den Stellen ab, an denen die Zonula-Fasern außen am Kapselsack angreifen. Dabei sind die Haptikteile gegenüber dem optischen Linsenteil nach vorne verschoben.

Aufgabe der Erfindung ist es, eine Korrekturlinse der eingangs genannten Art zu schaffen, bei der neben einer einwandfreien Positionierung und Fixierung, gegebenenfalls durch kapilare Haftung, gegenüber der natürlichen Augenlinse ein ausreichender Stoffwechsel an der natürlichen Augenlinse gewährleistet ist.

Diese Aufgabe wird erfindungsgemäß durch die kennzeichnenden Merkmale des Patentanspruches 1 gelöst.

Bei der Erfindung verläuft die Haptik, bestehend aus zwei Teilen, nämlich den den optischen Linsenteil umgebenden innenliegenden Haptikteil und den die Randzone bzw. außen liegenden Haptikteil bildenden Stützteil, in der Randzone an ihrer Rückseite senkrecht zur Linsenachse. Die Randzone der Haptik, d.h. der Stützteil, besitzt an seiner Rückseite und Vorderseite flache geradlinig und parallel zueinander verlaufende Oberflächen. Im implantierten Zustand der Linse wird dadurch erreicht, daß die Haptik in der Randzone von der Krümmung der natürlichen Augenlinse abhebend verläuft. Hierdurch erreicht man, daß auch im Falle einer kapillaren Haftung zwischen implantierter Korrekturlinse und vorderen Oberfläche der natürlichen Augenlinse die Haptik in der Randzone eine ständige abhebende Tendenz von der Auflagefläche zeigt. Hierdurch wird die Scheuerbelastung bei den natürlichen Bewegungen der Augenlinse auf die Zonula-Fasern erheblich gemindert. Der Gesamtdurchmesser der Korrekturlinse ist so bemessen, daß die Randzone der Haptik, d.h. der Stützteil, im Bereich der sich zwischen der natürlichen Augenlinse und dem Ziliarmuskel erstreckenden Zonula-Fasern aufliegt. Durch die waagerechte Führung der Randzone der Haptik (senkrechter Verlauf bezüglich der Linsenachse), deren Rückseite im wesentlichen dem radialen Verlauf der Zonula-Fasern angepaßt ist, gewährleistet eine sichere positionsbestimmende Auflage, wobei jedoch die Auflagefläche auf der natürlichen Augenlinse so gering wie möglich bemessen ist, um den Stoffwechsel nicht zu beeinträchtigen. Die Linse hat bevorzugt einen kreisrunden optischen Linsenteil, der von der zweiteiligen Haptik umgeben ist. Die zweiteilige Haptik hat im Sinne einer Linsenoberflächenverringerung von einer Kreisbogenform abweichende seitliche Begrenzungsränder an der Neun-Uhr- und Drei-Uhr-Seite. Die seitlichen Begrenzungsränder verlaufen parallel zueinander. Der obere und der untere Begrenzungsrand an der Zwölf-Uhr- und der Sechs-Uhr-Seite verlaufen in einer durchgehenden Kreisbogenform zwischen den jeweiligen beiden Enden der geradlinig verlaufenden seitlichen Begrenzungsränder. Die beiden Kreisbögen können einen gemeinsamen Mittelpunkt haben, der mit der optischen Achse der Linse zusammenfällt. ES handelt sich um Kreisbögen, die konzentrisch um den kreisförmigen optischen Linsenteil verlaufen, dessen Mittelpunkt ebenfalls in der optischen Achse der Linse (Linsenachse) liegt.

Zwischen der sich senkrecht zur Linsenachse erstreckenden Randzone der Haptik, die den Stützteil bildet, und dem optischen Linsenteil erstreckt sich als zweiter Haptikteil ein Positionierteil, dessen Vorderseite so gestaltet ist, daß in Zusammenwirkung mit der aufliegenden Iris die Positionierung der Korrekturlinse gegenüber der natürlichen Augenlinse in die gewünschte Lage erreicht wird. Hierbei sollen die optischen Achsen der natürlichen Linse und der Korrekturlinse in etwa zusammenfallen. Die Vorderseite des Positionierteils der Haptik bildet eine Gleitfläche für die Irisbewegung. Diese Vorderseite kann konkav, plan oder konvex verlaufen. In bevorzugterweise ist der Positionierteil so ausgebildet, daß er sich, beginnend von der kreisrunden Verbindungsstelle, mit dem optischen Linsenteil zum äußeren Rand der Korrekturlinse hin verjüngt.

Die Verbindungsstelle zwischen der Randzone (Stützteil) der Haptik und dem Positionierteil verläuft ebenfalls auf zwei Kreisbögen, die konzentrisch zum kreisrunden Rand des optischen Linsenteils und der beiden oberen und unteren kreisbogenförmigen Begrenzungsränder des Stützteils verlaufen. Die beiden Kreisbögen der Verbindungsstellen zwischen dem Positionierteil und dem Stützteil erstrecken sich ebenfalls durchgehend zwischen den beidseitigen Begrenzungsrändern.

Durch die gesamte geometrische Gestalt der Korrekturlinse wird im implantierten Zustand gewährleistet, daß die Oberfläche der natürlichen Augenlinse für den Stoffwechsel zugänglich bleibt. Zusätzlich kann die Haptik mit Durchbrüchen (Löchern) versehen sein, wodurch die von der Korrekturlinse abgedeckte Fläche der natürlichen Augenlinse noch zusätzlich verringert wird. Alle Begrenzungsränder der Linse können abgerundet sein, so daß die Linse keine scharfen Kanten aufweist.

Um eine möglichst schwimmende Lagerung des implantierten Linsenkörpers zu erzielen, besitzt er ein spezifisches Gewicht, das etwa gleich dem des Augenkammerwassers, nämlich etwa 1,1 ist.

Damit die Korrekturlinse nach der Implantation im Auge gegen Verdrehen gesichert ist, kann der jeweilige, im Bereich des Ziliar-Sulcus des Auges liegende Haptikumfangsteil einen unregelmäßigen bzw. unstetigen Kurvenverlauf haben, der jedoch nur unwesentlich von der Kreisform des Ziliar-Sulcus abweicht. Dabei wird ferner ein schonender Eingriff zwischen dem jeweiligen Haptikumfangsteil der Korrekturlinse und dem angrenzenden Augengewebe erreicht. Der unstetige Kurvenverlauf an den jeweiligen Haptikumfangsteilen kann poligonal ausgebildet sein oder konvexe und/oder konkave Verformungen aufweisen. Es können abwechselnd plane, konvexe oder konkave und auch kreisförmige Kontursegmente den jeweiligen Haptikumfangsteil bilden Die Tiefe bzw. Höhe der jeweiligen Verformungen, bezogen auf die benachbarten Umfangsteile, sind so bemessen, daß sie nur einen Bruchteil ihrer Längsausdehnung in Umfangsrichtung betragen. Hierdurch wird gewährleistet, daß beim Implantieren diese Verformungen, welche als Vorsprünge bzw. Ausnehmungen im Haptikummaterial in Erscheinung treten, ihre gewünschte Gestalt nicht verlieren und eine sichere Verdrehbremse für die implantierte Linse bilden. Dabei behält der Umfang eine an eine Kreisbogenrundform angenähte Gestalt bei.

In bevorzugter Weise kommen zwei, bezüglich der Linsenachse diametral angeordnete Haptikumfangsteile zur Anwendung, die im Bereich des Ziliar-Silcus des Auges nach der Implantation zu liegen kommen. Die Begrenzungslinien für die Kontursegmente an den Haptikumfangsteilen können plan oder kreisbogenförmig sein. In den Übergangsstellen zwischen unterschiedlich gestalteten Kontursegmenten, beispielsweise zwischen Plan und kreisbogenförmig gestalteten Kontursegmenten, ergeben sich Verankerungspunkte für das benachbarte Augengewebe, ohne daß dieses zu Entzündungen oder dergleichen gereizt wird. Diese Schnittpunkte gewährleisten die gewünschte Verdrehsicherheit der Korrekturlinse im Auge.

Anhand der Figuren wird an einem Ausführungsbeispiel die Erfindung noch näher erläutert. Es zeigt:
- Fig. 1: einen Schnitt durch den Linsenkörper eines ersten Ausführungsbeispiels;
- Fig. 2: eine Draufsicht auf den Linsenkörper der Fig. 1;
- Fig. 3: eine Draufsicht auf den Linsenkörper eines zweiten Ausführungsbeispiels;
- Fig. 4: eine Draufsicht auf en Linsenkörper eines dritten Ausführungsbeispiels;
- Fig. 5: eine Draufsicht auf den Linsenkörper eines vierten Ausführungsbeispiels;
- Fig. 6: in Draufsicht ein fünftes Ausführungsbeispiel;
- Fig. 7: in Draufsicht ein sechtes Ausführungsbeispiel;
- Fig. 8: in Draufsicht ein siebtes Ausführungsbeispiel;
- Fig. 9: in Draufsicht ein achtes Ausführungsbeispiel; und
- Fig. 10: in Draufsicht ein neuntes Ausführungsbeispiel.

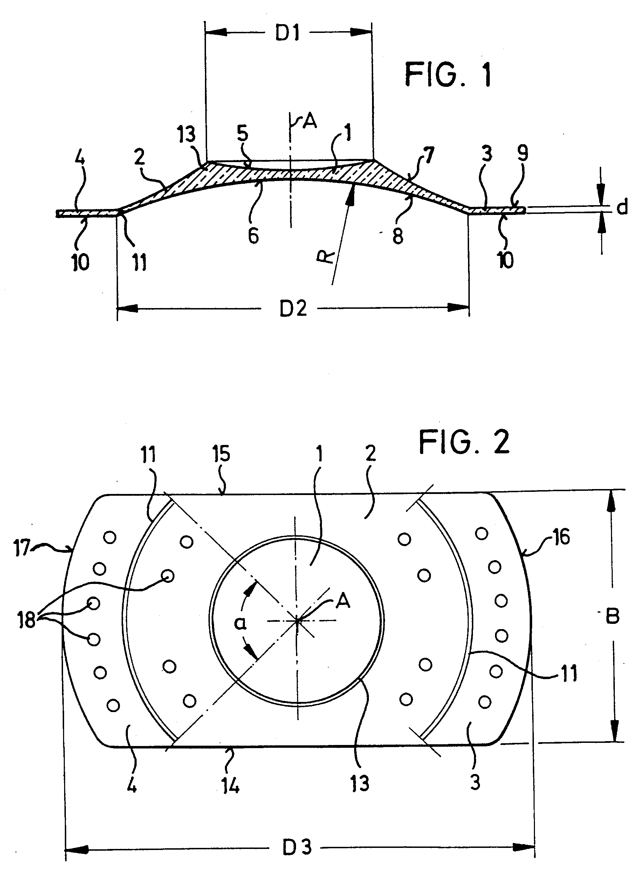

Bei dem in den Figuren 1 und 2 dargestellten Ausführungsbeispiel besteht der Linsenkörper der Korrekturlinse aus einem optischen Teil 1 und einer diesen optischen Teil umgebende zweiteilige Haptik. Die zweiteilige Haptik besteht aus einem sich an den optischen Linsenteil 1 anschließenden inneren Haptikteil bzw. Positionierteil 2 und einer Randzone (äußerer Haptikteil), die sich an den Positionierteil 2 als Stützteile 3 und 4 anschließt. Im dargestellten Ausführungsbeispiel ist der optische Linsenteil 1 kreisrund ausgebildet. Der optische Linsenteil 1 besitzt die optische Achse bzw. Linsenachse A. Der optische Linsenteil 1 bildet den Korrekturteil der Linse. Der optische Linsenteil 1 besitzt eine bikonkave Form mit einer Vorderseite 5 und einer Rückseite 6. Die Rückseite 6 besitzt einen Krümmungsradius, der an die Außenseite der natürlichen Augenlinse angepaßt ist. Der Krümmungsradius R der Rücksseite 6 des optischen Linsensteils 1 beträgt etwa 10 mm + 1 mm. Der Durchmesser D1 des optischen Linsenteils 1 beträgt 4 mm + 1 mm.

Der optische Linsenteil 1 geht in einer kreisrunden ringförmigen Verbindungsstelle 13 in die Haptik, und zwar den innen liegenden Teil der Haptik, nämlich dem Positionierteil 2, über. Der Querschnitt des Positionierteils 2 verjüngt sich, ausgehend von der Verbindungsstelle 13, nach außen hin, wie das insbesondere aus Fig. 1 zu ersehen ist. Beim dargestellten Ausführungsbeispiel ist der Positionierteil 2 bikonkav ausgebildet. Es ist jedoch auch möglich, daß der Positionierteil 2 plankonkav ausgebildet ist, wobei die Rückseite 8 des Positionierteils 2 den gleichen Krümmungsradius R aufweist wie die Rückseite 6 des optischen Linsenteils 1. Die Vorderseite 7 des Positionierteils 2 kann konkav oder plan ausgebildet sein. Es ist jedoch auch möglich, die Vorderseite 7 konvex auszubilden. Der Positionierteil 2 bildet eine Gleitfläche für die Irisbewegung, insbesondere für die Pupille. Die Ausgestaltung der Vorderseite 7 des Positionierteils (konkav, plan oder konvex) hängt von der Stärke des optischen Teiles 1 ab.

An den Positionierteil 2, welcher als einteilige Fläche den optischen Linsenteil 1 umfaßt, schließen sich nach außen hin zwei Randzonen der Haptik, nämlich die zwei Stützteile 3 und 4, an. Wie insbesondere aus Fig. 1 zu ersehen ist, weicht die Rückfläche 10 der Stützteile 3 und 4 erheblich von dem Verlauf der Rückflächen 6 und 8 des optischen Linsenteils und des Positionierteils ab. Die Rückflächen 10 sind plan ausgebildet und erstrecken sich im wesentlichen senkrecht zur Linsenachse A. Beim dargestellten Ausführungsbeispiel verlaufen auch die Vorderseiten 9 der Stützteile 3 und 4 senkrecht zur optischen Achse A und sind ebenfalls plan ausgebildet. Die Dicke d der Stützteile 3 und 4 beträgt etwa 0,1 mm. Verbindungsstellen 11 zwischen dem Positionierteil 2 und den Stützteilen 3 und 4 sind kreisbogenförmig ausgebildet. Die beiden Kreisbögen der Verbindungsstellen 11 haben einen gemeinsamen Mittelpunkt, der in der Linsenachse A liegt. Die kreisbogenförmigen Verbindungsstellen 11 enden an seitlichen Begrenzungsrändern 14 und 15. Die Begrenzungsränder 14 und 15 sind geradlinig ausgebildet. Beim dargestellten Ausführungsbeispiel erstrecken sich die kreisbogenförmigen Verbindungsstellen 11 innerhalb eines Winkelbereiches a von 90°. Dieser Winkelbereich kann je nach Breite B bzw. Abstand der beiden Begrenzungsränder 14 und 15 voneinander auch 80° bis 100° betragen. Beim dargestellten Ausführungsbeispiel hat der Linsenkörper eine Breite B von 6 mm + 1 mm.

Äußere Begrenzungsränder 16 und 17 der beiden Randzonen bzw. Stützteile 3 und 4 der Haptik liegen ebenfalls auf durchgehenden Kreisbögen, die an den Enden der seitlichen Begrenzungsränder 14 und 15 enden. Die beiden Kreisbögen der Begrenzungsränder 16 und 17 können ebenfalls einen gemeinsamen Mittelpunkt haben, der in der Linsenachse A liegt.

Der Gesamtdurchmesser D3 des Linsenkörpers, d.h. der Abstand der beiden kreisbogenförmigen Begrenzungsränder 16 und 17, voneinander beträgt beim dargestellten Ausführungsbeispiel 11 mm. Der Gesamtdurchmesser kann jedoch in Anpassung an das Auge, in welches die Korrekturlinse implantiert wird, geändert werden. Diese Änderungen liegen in der Regel in einem Bereich von + 1 mm.

Der Durchmesser D2 bzw. der Abstand der beiden kreisbogenförmigen Verbindungsstellen 11 beträgt beim dargestellten Ausführungsbeispiel 8 mm, d.h. etwa das doppelte des Durchmessers D1 des optischen Linsenteils 1. Der Durchmesser D2 kann in Abhängigkeit vom Durchmesser D3 ebenfalls variieren. Der Durchmesser D2 ist so bemessen, daß er weniger als drei Viertel des Durchmessers D3 beträgt.

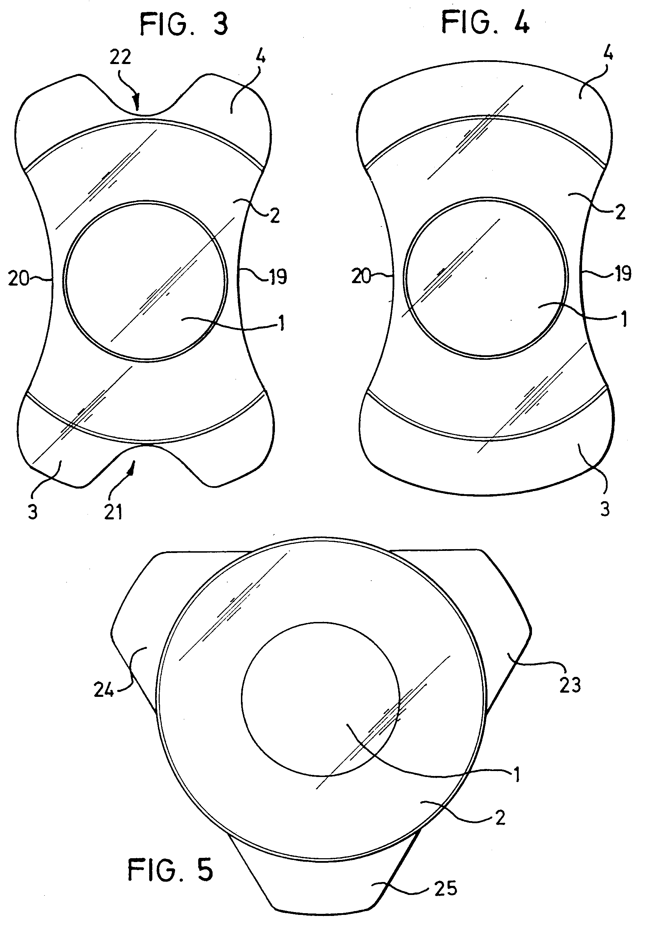

Bei den in den Figuren 3 und 4 dargestellten Ausführungsbeispielen sind die beiden seitlichen Begrenzungsränder 19 und 20 des Linsenkörpers bogenförmig gegebenenfalls kreisförmig nach innen (konkav) verlaufend geformt. Bei dem in der Figur 3 dargestellten Ausführungsbeispiel sind etwa in der Mitte der beiden Randzonen 3 und 4 Ausnehmungen 21 und 22 vorgesehen. Sowohl die konkav geformten seitlichen Begrenzungsränder19 und 20 als auch die Ausnehmungen 21 und 22 tragen aufgrund der Linsenoberflächenreduzierung zur Verbesserung des Stoffwechsels an der Außenseite der natürlichen Linse bei. Das in der Figur 5 dargestellte Ausführungsbeispiel besitzt einen sternförmigen Linsenkörper, wobei die äußere Haptik gebildet wird von den Vorsprüngen 23, 24 und 25, die zueinander einen Winkelabstand von 120° aufweisen. Der innere Haptikteil 2 wird von einem Kreisring gebildet.

Der aus der Haptik und dem optischen Teil bestehende Linsenkörper kann aus einem Stück gefertigt sein. Als Linsenmaterial eignet sich ein durchsichtiges, biokompatibles Material. Bevorzugt ist dieses Material als weiches und/oder elastisches Material ausgebildet. Ferner ist das Linsenmaterial in bevorzugter Weise hydrophil und/oder gasdurchlässig, insbesondere durchlässig für Sauerstoff, ausgebildet. Das Linsenmaterial kann aus Silikonkautschuk, Polyhydroxyethylmethacrylat, aus einem Copolymerisat von Silikon und Methylmethacrylat, Polyvinylpyrrolidon und anderen mit dem Augengewebe verträglichen Stoffen bestehen.

Der Linsenkörper kann im Bereich seiner Haptik ferner mit Durchbrüchen 18 (z.B. kreisrunden Löchern oder ähnlichem) ausgestattet sein. Hierdurch wird die Auflagefläche der Korrekturlinse weiterhin verringert, und die Oberfläche der natürlichen Augenlinse ist für den Stoffwechsel in erhöhtem Maße zugänglich.

In bevorzugter Weise kommt die Korrekturlinse bei starker Myopie zum Einsatz. Hierzu ist der optische Linsenteil 1 als Negativlinse ausgebildet. Anstelle der bikonkaven Gestalt des optischen Linsenteils kann dieser auch eine konkavkonvexe oder torische Form aufweisen.

Bei der Implantation wird die Augenpupille erweitert, so daß die Korrekturlinse in ihrer Ausdehnung des Durchmessers D3 zwischen die Iris und die Außenoberfläche der natürlichen Augenlinse geschoben werden kann. Der optische Linsenkörper 1 kann dann mit seiner Linsenachse A mit der optischen Achse der natürlichen Linse ausgerichtet werden. Die Randzonen bzw. Stützteile 3 und 4 der Haptik kommen dann im Bereicht der Zonula-Fasern zu liegen. Aufgrund der speziellen Ausbildung dieser Randzonen ist die natürliche Bewegung dieser Fasern nicht oder kaum behindert.

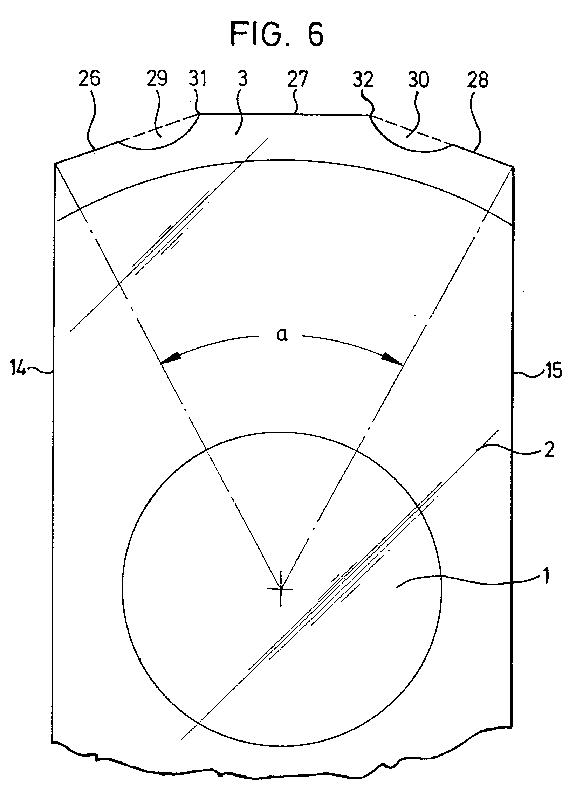

Die in den Figuren 6 bis 10 dargestellten Ausführungsbeispiele (Positionierteil 2, Stützteile 3) besitzen den optischen Linsenteil 1, der von der Haptik umgeben ist. Die Haptik besitzt bei den dargestellten Ausführungsbeispielen die im wesentlichen geradlinig verlaufenden seitlichen Linsenränder 14 und 15. Es ist jedoch auch möglich, daß diese seitlichen Linsenränder konkav nach innen gebogen sind. Die Haptik besitzt zwei Haptikumfangsteile - in den Figuren 1 bis 5 ist jeweils ein Haptikumfangsteil gezeigt -, welche mit den Stützteilen (Stützteil 3 ist dargestellt) im implantierten Zustand im Bereichdes Ziliar-Sulcus liegen. Bezüglich der Linsenachse A (optische Achse der Linse) als Scheitelpunkt erstrecken sich die beiden Haptikumfangsteile in einem Winkelbereich a. Dieser Winkel a kann in der Größenordnung von 50° bis 90° liegen.

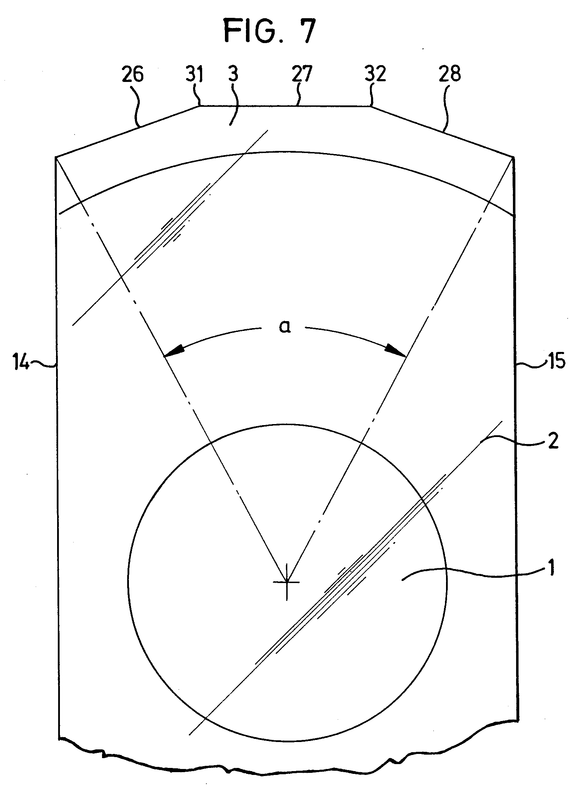

Bei den dargestellten Ausführungsbeispielen besitzen die Haptikumfangsteile einen unstetigen Kurvenverlauf des Umfangsrades. Bei den Ausführungsbeispielen der Figuren 6 und 7 ist der Kurvenverlauf des Haptikumfangs innerhalb des Winkelbereichs a ein Poligon. Beim Ausführungsbeispiel der Figur 7 ist es ein reines Poligon, das aus drei Poligonseiten 26, 27 und 28 besteht. Bei dem Ausführungsbeispiel der Figur 6 sind in den Poligonseiten 26 und 28, d.h. in beiden äußeren Poligonseiten konkave Einbuchgungen 29 und 30 vorgesehen. Diese konkaven Einbuchtungen sind so angeordnet, daß ihre einen Enden (inneren Enden) an Poligonschnittpunkten 31, 32 der Poligonseiten 26, 28 mit der Poligonseite 27 liegen. Die konkaven Einbuchtungen 29 und 30 können jedoch auch in der Mitte oder an einer anderen Stelle im Bereich der Poligonseiten 26 und 28 angeordnet sein.

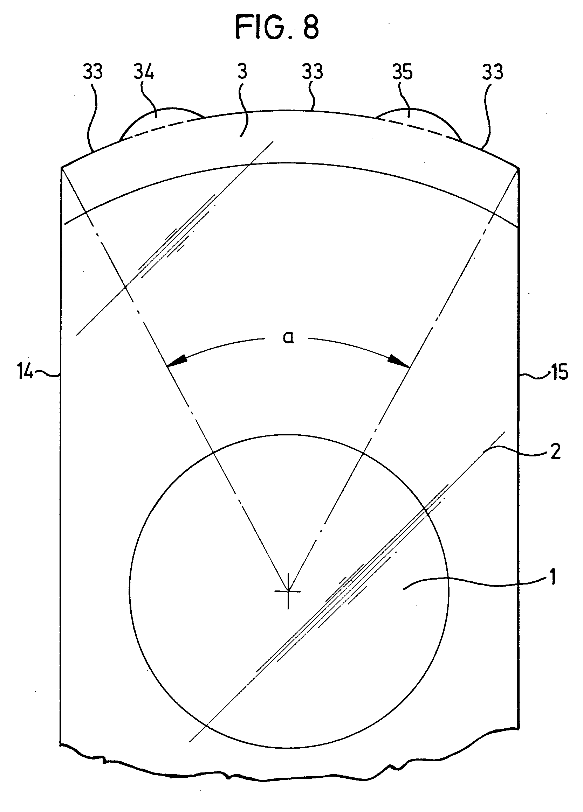

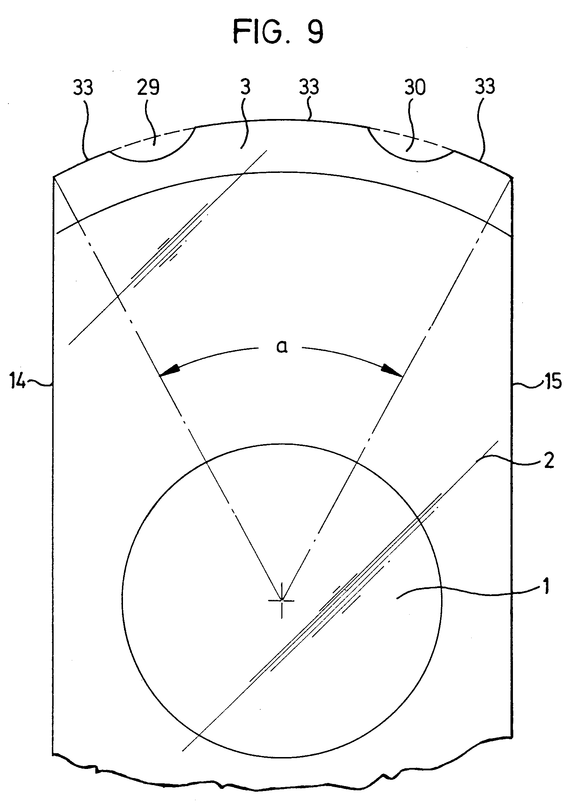

Bei dem in der Figur 8 dargestellten Ausführungsbeispiel besitzt der Haptikumfangsteil innerhalb des Winkelbereichs a einen im wesentlichen kreisbogenförmigen Verlauf mit konvexen Vorsprüngen 34 und 35 und konkaven Einbuchtungen 29 und 30 beim Ausführungsbeispiel der Figur 9.

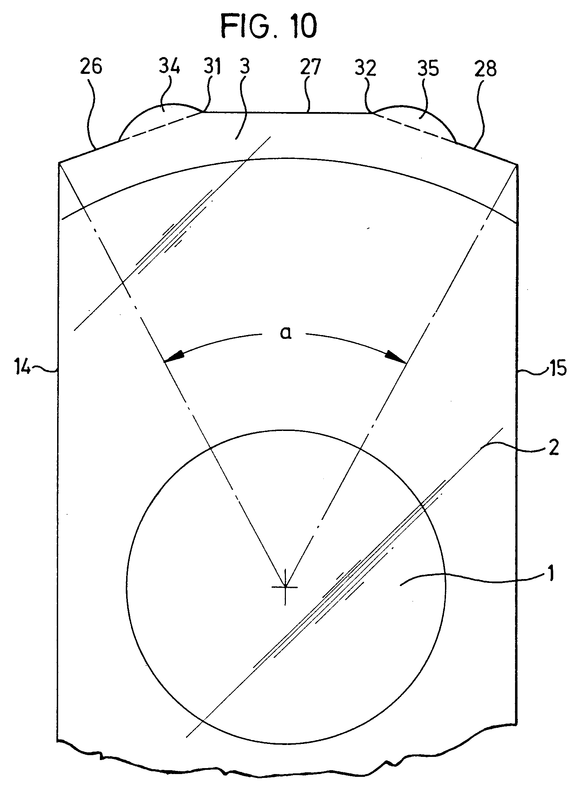

Bei dem in der Figur 10 dargestellten Ausführungsbeispiel hat der Haptikumfangsteil einen poligonen Verlauf, der sich aus den Poligonseiten 26, 27 und 28 zusammensetzt, die sich in Schnittpunkten 31 und 32 schneiden. Im Bereich der Poligonseiten 26 und 28 sind konvexe Verformungen bzw. Erhöhungen 34 und 35 gegenüber dem poligonen Umfangsverlauf vorgesehen. Die beiden inneren Enden der konvexen Erhöhungen 34 und 35 liegen an den Stellen der Schnittpunkte 31 und 32 der Poligonseiten 26 und 28 mit der Poligonseite 27.

Die Tiefe der konkaven Einbuchtungen 29 und 30 in den Ausführungsformen der Figuren 6 und 9 gegenüber dem benachbarten Umfangsbereich beträgt 0,2 bis 0,4 mm. Beim Ausführungsbeispiel der Figur 6 ist die Tiefe bezogen auf die Fortsetzung (strichliert dargestellt) der Poligonseiten im Bereich der Einbuchtungen 29 und 30. Beim Ausführungsbeispiel der Figur 9 ist die Tiefe der Einbuchtungen bezogen auf die Fortsetzung (strichliert dargestellt) des kreisbogenförmigen Umfangrandes im Bereich der Einbuchtungen 29 und 30.

Bei den in den Figuren 8 und 10 dargestellten Ausführungsbeispielen ist die Höhe der konvexen Verformungen bzw. Vorsprünge 34 und 35 bezogen auf die Fortsetzung (strichliert dargestellt) der benachbarten Kreisbogensegmente 33 im Bereich der Vorsprünge 34 und 35 (Figur 8) bzw. bezogen auf die Fortsetzung (strichliert dargestellt) der Poligonseiten 26, 27, 28 im Bereich der Vorsprünge 34 und 35 (Fig. 10).

Die Längenausdehnung in Umfangsrichtung der konkaven Einbuchtungen 29 und 30 und konvexen Verformungen 34 und 35 in den Ausführungsbeispielen der Figuren 6 und 8 bis 10 beträgt 0,8 bis 1,4 mm.

Bei den in den Figuren 6 bis 10 dargestellten Ausführungsbeispielen bleibt der unstetige Kurvenverlauf des Haptikumfangsteils angenähert an eine Kreisbogenform. Diese Kreisbogenform hat einen Durchmesser, der angepaßt ist an den Innendurchmesser des Ziliar-Sulcus des Auges, in welches die Linse implantiert werden soll. In der Regel liegen diese Durchmesser im Bereich von 11,0 bis 13,5 mm. Die die Unstetigkeit des Kurvenverlaufs erzeugenden Konturverformungen in Form der Einbuchtungen 29 und 32 bzw. der Vorsprünge 34 und 35 sowie die Übergangsstellen zwischen diesen Verformungen und den übrigen Teilen des Umfangsverlaufs (Poligonseiten 26 bis 28, bzw. Kreisbögen 33) vermitteln eine Verdrehbremse, durch die gewährleistet ist, daß die Linse um ihre optische Achse A verdrehsicher im Auge implantiert bleibt. Dies gilt auch für die in der Figur 7 dargestellte Ausführungsform, bei der die Umfangskontur ein reines Poligon ist. Dieses Poligon besitzt beim dargestellten Ausführungsbeispiel drei Poligonseiten 26, 27 und 28. Es ist jedoch auch möglich, mehr Poligonseiten zu verwenden. Die Schnittpunkte 31, 32 der Poligonseiten gewährleisten die erforderliche Sicherheit gegen Verdrehen.

Bei den Ausführungsformen der Figuren 6, 7 und 10, bei denen die Ausgangskontur des Umfangs ein Poligon ist, ergibt sich noch der Vorteil, daß der Linsenkörper aus einem größeren Linsenkörper als Grundform auf den Durchmesser des Ziliar-Sulcus des Auges, in das die Linse implantiert werden soll, durch Zuschnitt angepaßt werden kann. Es läßt sich damit eine exakte Anpassung des Gesamtdurchmessers der diametral einander gegenüber liegenden beiden Haptikumfangsteile an das Auge erreichen.

Bei den in den Figuren dargestellten Ausführungsbeispielen hat der optische Linsenteil 1 in der Regel einen Durchmesser von 4,0 mm. Die Breite der Haptik 2 von einer Seitenkante 14 zur anderen Seitenkante 15 beträgt ca. 6,0 mm.

Wenn es sich um eine Hochmyopielinse mit ca. -50 dpt handelt, beträgt der Durchmesser des optischen Linsenteils ca. 2,0 mm. Die in den Figuren 6 bis 10 dargestellten Ausführungsbeispiele können einen Querschnitt aufweisen, wie er in der Figur 1 dargestellt ist. Natürlich eignen sich die in den Figuren 6 bis 10 dargestellten Ausführungsformen auch für Myopielinsen mit anders ausgebildeten Haptiken, z.B. Haptiken, die nicht in zwei Bereiche unterteilt sind. Auch dann wird die Verdrehsicherheit durch die gezeigten Randformen erreicht.

## Patentansprüche

1. Korrekturlinse, welche auf die Vorderseite einer natürlichen Linse eines Auges implantierbar ist, mit einem optischen Linsenteil (1) und einer den optischen Linsensteil umgebenden Haptik (2, 3, 4), die in radialer Richtung in einen den optischen Linsenteil (1) umgebenden inneren Haptikteil (2) und einen äußeren Haptikteil (3, 4; 23, 24, 25), deren äußere Begrenzungslinien (11, 16, 17) zumindest teilweise auf Kreisbögen liegen, unterteilt ist, wobei der äußere Haptikteil (3, 4; 23, 24, 25) an seiner der natürlichen Linse zugewandten Rückseite (10) einen vom Verlauf der Oberflächengeometrie der Rückflächen (6, 8) von optischem Linsenteil (1) und innerem Haptikteil (2) abweichenden geometrischen Verlauf aufweist,
dadurch **gekennzeichnet,**
daß der äußere Haptikteil (3, 4; 23, 24, 25) beginnend an der äußeren Begrenzungslinie (11) des inneren Haptikteils (2) zum äußeren Rand (16, 17) des äußeren Haptikteils (3, 4; 23, 24, 25) hin und seine Rückseite (10) sich senkrecht zur optischen Achse (A) des optischen Linsenteils (1) erstrecken.

2. Korrekturlinse nach Anspruch 1, dadurch gekennzeichnet, daß der äußere Haptikteil (3, 4; 23, 24, 25) mehrteilig ausgebildet ist.

3. Korrekturlinse nach Anspruch 1 oder 2, dadurch gekennzeichnet, daß der Querschnitt des inneren Haptikteils (2), beginnend von einer äußeren Begrenzungslinie (13) des optischen Linsenteils (1) zur äußeren Begrenzungslinie (11) des inneren Haptikteils (2) hin sich verjüngt.

4. Korrekturlinse nach einem der Ansprüche 1 bis 3, dadurch gekennzeichnet, daß in der Sechs-Uhr-/Zwölf-Uhr-Richtung des Linsenkörpers (1, 2, 3, 4) die Ausdehnung des optischen Linsenteils (1) und des inneren Haptikteils (2) weniger als drei Viertel der Gesamtausdehnung des Linsenkörpers in dieser Ausdehnungsrichtung betragen.

5. Korrekturlinse nach einem der Ansprüche 1 bis 4, dadurch gekennzeichnet, daß in der Sechs-Uhr-/Zwölf-Uhr-Richtung des Linsenkörpers (1, 2, 3, 4) der Durchmesser des kreisrunden optischen Linsenteils (1) etwa die Hälfte des Durchmessers (D2) des inneren Haptikteils (2) in dieser Ausdehnungsrichtung beträgt.

6. Korrekturlinse nach einem der Ansprüche 1 bis 5, dadurch gekennzeichnet, daß der Linsenkörper (1, 2, 3, 4) an der Neun-Uhr- und der Drei-Uhr-Seite einen von einer Kreisbogenform zum Innern des Kreises abweichenden Verlauf aufweist.

7. Korrekturlinse nach Anspruch 6, dadurch gekennzeichnet, daß der Linsenkörper (1, 2, 3, 4) an der Neun-Uhr und der Drei-Uhr-Seite parallele geradlinig verlaufende Begrenzungsränder (14, 15) aufweist.

8. Korrekturlinse nach Anspruch 6, dadurch gekennzeichnet, daß der Linsenkörper an der Neun-Uhr- und der Drei-Uhr-Seite konkav verlaufende Begrenzungsränder aufweist.

9. Korrekturlinse nach einem der Ansprüche 1 bis 5, dadurch gekennzeichnet, daß der Linsenkörper sternförmig ausgebildet ist.

10. Korrekturlinse nach einem der Ansprüche 1 bis 8, dadurch gekennzeichnet, daß an der Zwölf-Uhr-Seite und der Sechs-Uhr-Seite des Linsenkörpers (1, 2, 3, 4) die Begrenzungsränder (16, 17) des äußeren Haptikteils 2 entlang von durchgehenden sich von einem seitlichen Begrenzungsrand (14) bis zum anderen seitlichen Begrenzungsrand (15) erstreckenden Kreisbögen verlaufen.

11. Korrekturlinse nach Anspruch 10, dadurch gekennzeichnet, daß der Mittelpunkt der Kreisbögen in der Linsenachse (A) liegt.

12. Korrekturlinse nach einem der Ansprüche 1 bis 8, dadurch gekennzeichnet, daß die Verbindungsstellen zwischen dem inneren Haptikteil (2) und dem äußeren Haptikteil (3, 4) auf zwei Kreisbögen liegen, deren gemeinsamer Mittelpunkt in der Linsenachse (A) liegt.

13. Korrekturlinse nach Anspruch 12, dadurch gekennzeichnet, daß sich jeder der beiden Kreisbögen innerhalb eines Winkels, der 80° bis 100°, insbesondere 90°, beträgt, erstreckt.

14. Korrekturlinse nach einem der Ansprüche 1 bis 10, dadurch gekennzeichnet, daß die Begrenzungsränder (14, 15, 16 17) abgerundet sind.

15. Korrekturlinse nach einem der Ansprüche 1 bis 14, da durch gekennzeichnet, daß die Begrenzungsränder (16, 17) des äußeren Haptikteils (3, 4) konkave Ausnehmungen (21, 22) aufweisen.

16. Korrekturlinse nach einem der Ansprüche 1 bis 15, dadurch gekennzeichnet, daß die Vorderseite (9) und die Rückseite (10) der äußeren Haptikteile (3, 4) von der Verbindungsstelle (11) mit dem inneren Haptikteil (2) bis zum äußeren Begrenzungsrand (16, 17) der äußeren Haptikteile plan ausgebildet sind und parallel zueinander verlaufen.

17. Korrekturlinse nach einem der Ansprüche 1 bis 16, dadurch gekennzeichnet, daß im jeweiligen äußeren Haptikteil (3, 4) und/oder im inneren Haptikteil (2) Durchbrüche (18) vorgesehen sind.

18. Korrekturlinse nach einem der Ansprüche 1 bis 17, dadurch gekennzeichnet, daß der jeweilige, im Bereich des Ziliar-Sulcus des Auges liegende Haptikumfangsteil (26 - 28; 33) einen unstetigen Kurvenverlauf hat.

19. Korrekturlinse nach Anspruch 18, dadurch gekennzeichnet, daß der jeweilige Haptikumfangsteil (26 - 28) poligonal ausgebildet ist.

20. Korrekturlinse nach Anspruch 18 oder 19, dadurch gekennzeichnet, daß der jeweilige Haptikumfangsteil (26 - 28; 33) konkave und/oder konvexe Verformungen (29, 30; 34, 35) aufweist, deren jeweilige Tiefe beziehungsweise Höhe bezogen auf die benachbarten Umfangsbereiche ein Bruchteil ihrer Längenausdehnung in Umfangsrichtung beträgt.

21. Korrekturlinse nach Anspruch 19 oder 20, dadurch gekennzeichnet, daß das jeweils eine Ende der konkaven bzw. konvexen Verformungen (29, 30; 34, 35) an jeweiligen Poligonschnittpunkten (31, 32) angeordnet sind.

22. Korrekturlinse nach einem der Ansprüche 19 bis 21, dadurch gekennzeichnet, daß der jeweilige Haptikumfangsteil (26 - 28) drei Poligonseiten aufweist.

23. Korrekturlinse nach einem der Ansprüche 18 bis 22, dadurch gekennzeichnet, daß die konkaven bzw. konvexen Verformungen (29, 30; 34, 35) eine Tiefe bzw. Höhe bezüglich der benachbarten Umfangsbereiche besitzen, welche einem Sechstel bis einem Drittel ihrer Längsausdehnung in Umfangsrichtung entspricht.

24. Korrekturlinse nach einem der Ansprüche 20 bis 23, dadurch gekennzeichnet, daß die konkaven bzw. konvexen Verformungen (29, 30; 34, 35) kreisbogenförmige Begrenzungslinien aufweisen.

25. Korrentkurlinse nach Anspruch 20 oder 23, dadurch gekennzeichnet, daß die konkaven bzw. konvexen Verformungen (29, 30; 34, 35) eine Tiefe bzw. Höhe von 0,2 - 0,4 mm und eine Längsausdehnung von 0,8 - 1,4 mm in Umfangsrichtung haben.

26. Korrekturlinse nach einem der Ansprüche 1 bis 25, dadurch gekennzeichnet, daß der optische Linsenteil (1) als Konkavlinse ausgebildet ist.

27. Korrekturlinse nach Anspruch 26, dadurch gekennzeichnet, daß die Konkavlinse einen Durchmesser von ca. 2,0 mm hat.

## Claims

1. A correction lens which can be implanted onto the front of a natural lens of an eye, comprising an optical lens portion (1) and a haptic (2, 3, 4) which surrounds the optical lens portion and which is subdivided in the radial direction into an inner haptic part (2) surrounding the optical lens portion (1) and an outer haptic part (3, 4; 23, 24, 25) whose outer boundary lines (11, 16, 17) lie at least partially on circular arcs, wherein the outer haptic part (3, 4; 23, 24, 25), at its rear side (10) towards the natural lens, is of a geometrical configuration which differs from the configuration of the surface geometry of the rear surfaces (6, 8) of the optical lens portion (1) and the inner haptic part (2), characterised in that the outer haptic part (3, 4; 23, 24, 25), beginning at the outer boundary line (11) of the inner haptic part (2) towards the outer edge (16, 17) of the outer haptic part (3, 4; 23, 24, 25) and its rear side (10) extend perpendicularly to the optical axis (A) of the optical lens portion (1).

2. A correction lens according to claim 1 characterised in that the outer haptic part (3, 4; 23, 24, 25) is of a multi-part nature.

3. A correction lens according to claim 1 or claim 2 characterised in that the cross-section of the inner haptic part (2) tapers beginning from an outer boundary line (13) of the optical lens portion (1) towards the outer boundary line (11) of the inner haptic part (2).

4. A correction lens according to one of claims 1 to 3 characterised in that in the six o'clock/twelve o'clock direction of the lens body (1, 2, 3, 4) the extent of the optical lens portion (1) and the inner haptic part (2) is less than three quarters of the total extent of the lens body in said direction of extent.

5. A correction lens according to one of claims 1 to 4 characterised in that in the six o'clock/twelve o'clock direction of the lens body (1, 2, 3, 4) the diameter of the circular optical lens portion (1) is approximately half the diameter (D2) of the inner haptic part (2) in said direction of extent.

6. A correction lens according to one of claims 1 to 5 characterised in that at the nine o'clock and the three o'clock sides the lens body (1, 2, 3, 4) is of a configuration which differs from a circular arc shape towards the interior of the circle.

7. A correction lens according to claim 6 characterised in that at the nine o'clock and the three o'clock sides the lens body (1, 2, 3, 4) has parallel rectilinearly extending boundary edges (14, 15).

8. A correction lens according to claim 6 characterised in that at the nine o'clock and the three o'clock sides the lens body has concavely extending boundary edges.

9. A correction lens according to one of claims 1 to 5 characterised in that the lens body is of a star configuration.

10. A correction lens according to one of claims 1 to 8 characterised in that at the twelve o'clock side and the six o'clock side of the lens body (1, 2, 3, 4) the boundary edges (16, 17) of the outer haptic part (2) extend along continuous circular arcs extending from one lateral boundary edge (14) to the other lateral boundary edge (15).

11. A correction lens according to claim 10 characterised in that the centre point of the circular arcs is on the lens axis (A).

12. A correction lens according to one of claims 1 to 8 characterised in that the connecting locations between the inner haptic part (2) and the outer haptic part (3, 4) are on two circular arcs whose common centre point is on the lens axis (A).

13. A correction lens according to claim 12 characterised in that each of the two circular arcs extends within an angle which is 80° to 100°, in particular 90°.

14. A correction lens according to one of claims 1 to 10 characterised in that the boundary edges (14, 15, 16, 17) are rounded.

15. A correction lens according to one of claims 1 to 14 characterised in that the boundary edges (16, 17) of the outer haptic part (3, 4) have concave recesses (21, 22).

16. A correction lens according to one of claims 1 to 15 characterised in that the front side (9) and the rear side (10) of the outer haptic parts (3, 4) are flat and extend in mutually parallel relationship from the connecting location (11) to the inner haptic part (2) to the outer boundary edge (16, 17) of the outer haptic parts.

17. A correction lens according to one of claims 1 to 16 characterised in that openings (18) are provided in the respective outer haptic part (3, 4) and/or in the inner haptic part (2).

18. A correction lens according to one of claims 1 to 17 characterised in that the respective peripheral haptic part (26 - 28; 33) which is in the region of the ciliary sulcus of the eye is of a non-uniform curve configuration.

19. A correction lens according to claim 18 characterised in that the respective peripheral haptic part (26 - 28) is of a polygonal configuration.

20. A correction lens according to claim 18 or claim 19 characterised in that the respective peripheral haptic part (26 - 28; 33) has concave and/or convex shaped portions (29, 30; 34, 35) whose respective depth or height with respect to the adjacent peripheral regions is a fraction of the longitudinal extent thereof in the peripheral direction.

21. A correction lens according to claim 19 or claim 20 characterised in that the one end of each of the concave or convex shaped portions (29, 30; 34, 35) is respectively arranged at the respective polygon intersections (31, 32).

22. A correction lens according to one of claims 19 to 21 characterised in that the respective peripheral haptic part (26 - 28) has three sides of a polygon.

23. A correction lens according to one of claims 18 to 22 characterised in that the concave or convex shaped portions (29, 30; 34, 35) are of a depth or height with respect to the adjacent peripheral regions, which corresponds to a sixth to a third of the longitudinal extent thereof in the peripheral direction.

24. A correction lens according to one of claims 20 to 23 characterised in that the concave or convex shaped portions (29, 30; 34, 35) have boundary lines in the form of circular arcs.

25. A correction lens according to claim 20 or claim 23 characterised in that the concave or convex shaped portions (29, 30; 34, 35) are of a depth or height of 0.2 - 0.4 mm and a longitudinal extent of 0.8 - 1.4 mm in the peripheral direction.

26. A correction lens according to one of claims 1 to 25 characterised in that the optical lens part (1) is in the form of a concave lens.

27. A correction lens according to claim 26 characterised in that the concave lens is of a diameter of about 2.0 mm.

## Revendications

1. Lentille correctrice implantable sur la face antérieure du cristallin, comportant une partie optique (1) et une haptique (2, 3, 4) qui entoure cette partie optique et est divisée dans la direction radiale en une partie intérieure (2) entourant la partie optique (1) de la lentille et une partie extérieure (3, 4 ; 23, 24, 25) dont les lignes limites extérieures (11, 16, 17) sont situées au moins en partie sur des arcs de cercle, la partie extérieure (3, 4 ; 23, 24, 25) de l'haptique ayant sur sa face postérieure (10) dirigée vers le cristallin une forme qui diffère de celle des faces postérieures (6, 8) de la partie optique (1) de la lentille et de la partie intérieure (2) de l'haptique,
caractérisée par le fait que
la partie extérieure (3, 4 ; 23, 24, 25) de l'haptique s'étend à partir de la ligne limite extérieure (11) de la partie intérieure (2) de l'haptique vers le bord extérieur (16, 17) de la partie extérieure (3, 4 ; 23, 24, 25) de l'haptique et sa face postérieure (10) s'étend perpendiculairement à l'axe optique (A) de la partie optique (1) de la lentille.

2. Lentille correctrice selon la revendication 1, caractérisée par le fait que la partie extérieure (3, 4 ; 23, 24, 25) de l'haptique est en plusieurs parties.

3. Lentille correctrice selon l'une des revendications 1 et 2, caractérisée par le fait que la section transversale de la partie intérieure (2) de l'haptique diminue de la ligne limite extérieure (13) de la partie optique (1) de la lentille vers la ligne limite extérieure (11) de la partie intérieure (2) de l'haptique.

4. Lentille correctrice selon l'une des revendications 1 à 3, caractérisée par le fait que dans là direction six heures/douze heures du corps de lentille (1, 2, 3, 4), l'étendue de la partie optique (1) de la lentille et de la partie intérieure (2) de l'haptique est inférieure aux trois quarts de l'étendue totale du corps de lentille dans cette direction.

5. Lentille correctrice selon l'une des revendications 1 à 4, caractérisée par le fait que dans la direction six heures/douze heures du corps de lentille (1, 2, 3, 4), le diamètre de la partie optique circulaire (1) de la lentille est à peu près égal à la moitié du diamètre (D2) de la partie intérieure (2) de l'haptique dans cette direction.

6. Lentille correctrice selon l'une des revendications 1 à 5, caractérisée par le fait que le corps de lentille (1, 2, 3, 4) a sur le côté neuf heures et le côté trois heures une forme qui diffère d'une forme en arc de cercle vers l'intérieur du cercle.

7. Lentille correctrice selon la revendication 6, caractérisée par le fait que le corps de lentille (1, 2, 3, 4) présente sur le côté neuf heures et le côté trois heures des bords limites rectilignes parallèles (14, 15).

8. Lentille correctrice selon la revendication 6, caractérisée par le fait que le corps de lentille présente sur le côté neuf heures et le côté trois heures des bords limites concaves.

9. Lentille correctrice selon l'une des revendications 1 à 5, caractérisée par le fait que le corps de lentille est en forme d'étoile.

10. Lentille correctrice selon l'une des revendications 1 à 8, caractérisée par le fait que sur le côté douze heures et le côté six heures du corps de lentille (1, 2, 3, 4), les bords limites (16, 17) de la partie extérieure (2) de l'haptique suivent des arcs de cercle continus s' étendant d'un bord limite latéral (14) à l'autre bord limite latéral (15).

11. Lentille correctrice selon la revendication 10, caractérisée par le fait que le centre des arcs de cercle est situé sur l'axe (A) de la lentille.

12. Lentille correctrice selon l'une des revendications 1 à 8, caractérisée par le fait que les endroits de raccordement entre la partie intérieure (2) de l'haptique et la partie extérieure (3, 4) de l'haptique se trouvent sur deux arcs de cercle dont le centre commun est situé sur l'axe (A) de la lentille.

13. Lentille correctrice selon la revendication 12, caractérisée par le fait que chacun des deux arcs de cercle s'étend sur un angle de 80° à 100°, en particulier de 90°.

14. Lentille correctrice selon l'une des revendications 1 à 10, caractérisée par le fait que les bords limites (14, 15, 16, 17) sont arrondis.

15. Lentille correctrice selon l'une des revendications 1 à 14, caractérisée par le fait que les bords limites (16, 17) de la partie extérieure (3, 4) de l'haptique présentent des évidements concaves (21, 22).

16. Lentille correctrice selon l'une des revendications 1 à 15, caractérisée par le fait que la face antérieure (9) et la face postérieure (10) des parties extérieures (3, 4) de l'haptique sont planes et s'étendent parallèlement de l'endroit de raccordement (11) à la partie intérieure (2) de l'haptique jusqu'au bord limite extérieur (16, 17) des parties extérieures de l'haptique.

17. Lentille correctrice selon l'une des revendications 1 à 16, caractérisée par le fait que dans chacune des parties extérieures (3, 4) de l'haptique et/ou dans la partie intérieure (2) de l'haptique sont prévues des ouvertures (18).

18. Lentille correctrice selon l'une des revendications 1 à 17, caractérisée par le fait que chaque partie périphérique (26 à 28 ; 33) de l'haptique située dans la zone du sillon ciliaire de l'oeil a une allure courbe discontinue.

19. Lentille correctrice selon la revendication 18, caractérisée par le fait que chaque partie périphérique (26 à 28) de l'haptique est polygonale.

20. Lentille correctrice selon l'une des revendications 18 et 19, caractérisée par le fait que chaque partie périphérique (26 à 28 ; 33) de l'haptique présente des déformations concaves et/ou convexes (29, 30 ;34, 35) dont la profondeur ou la hauteur relativement aux zones périphériques voisines est égale à une fraction de leur étendue longitudinale dans la direction circonférentielle.

21. Lentille correctrice selon l'une des revendications 19 et 20, caractérisée par le fait qu'une extrémité des déformations concaves ou convexes (29, 30 : 34, 35) est située à un sommet (31, 32) du polygone.

22. Lentille correctrice selon l'une des revendications 19 à 21, caractérisée par le fait que chaque partie périphérique (26 à 28) de l'haptique présente trois côtés de polygone.

23. Lentille correctrice selon l'une des revendications 18 à 22, caractérisée par le fait que les déformations concaves ou convexes (29, 30 ; 34, 35) ont une profondeur ou une hauteur relativement aux zones périphériques voisines qui est comprise entre le sixième et le tiers de leur étendue longitudinale dans la direction circonférentielle.

24. Lentille correctrice selon l'une des revendications 20 à 23, caractérisée par le fait que les déformations concaves ou convexes (29, 30 ; 34, 35) présentent des lignes limites en arc de cercle.

25. Lentille correctrice selon l'une des revendications 20 et 23, caractérisée par le fait que les déformations concaves ou convexes (29, 30 ; 34, 35) ont une profondeur ou une hauteur de 0,2 à 0,4 mm et une étendue longitudinale de 0,8 à 1,4 mm dans la direction circonférentielle.

26. Lentille correctrice selon l'une des revendications 1 à 25, caractérisée par le fait que la partie optique (1) de la lentille est une lentille concave.

27. Lentille correctrice selon la revendication 26, caractérisée par le fait que le diamètre de la lentille concave est d'environ 2,0 mm.
